# EUROPEAN PATENT APPLICATION

(11) **EP 4 202 427 A1**
(43) Date of publication of application: **28.06.2023**
(21) Application number: 21217638.2
(22) Date of filing: 23.12.2021
(51) Int. Cl.: G01N 24/08, G01R 33/56, G01R 33/561, G06T 7/00, B07C 5/344, G01N 33/08, G01R 33/483

(54) **DIRECT INFERENCE BASED ON UNDERSAMPLED MRI DATA OF INDUSTRIAL SAMPLES**

(71) Applicant: Orbem GmbH, 85748 Garching b.Munich (DE)
(72) Inventor: Coello Uribe, Jorge Eduardo, 80802 München (DE); Kudielka, Guido, 71384 Weinstadt (DE); Gómez Damián, Pedro Agustín, 80802 München (DE); Laparidou, Maria, 80634 München (DE); Molina Romero, Miguel, 80939 München (DE)
(74) Representative: Pelster Behrends Patentanwälte PartG mbB

(57) **Abstract**

Method for automated non-invasive identification of a predetermined feature in a multitude of industrial samples (102) of a predefined sample type, the method comprising the steps of: a) conveying an industrial sample (102) of the predefined sample type into an MRI scanner (106), b) recording in an MRI measurement for at least one slice or at least a partial volume of the industrial sample undersampled MRI data (300), comprising: - undersampled raw MRI data, comprising a multitude of time dependent signals for different phases, and/or - processed MRI data, obtained from processing undersampled raw MRI data, and c) analysing the undersampled MRI data (300) with an inference module (200) for identifying a predetermined feature of the industrial sample (102) using a machine learning module (204) that is trained for identifying the predetermined feature in industrial samples (102) of the predefined sample type from undersampled MRI data (300), wherein the inference module (200) comprises a memory (202) storing the machine learning module (204) and a processor (206) for controlling the inference module (200), wherein the inference module (200) is configured to provide the undersampled MRI data (300) as an input to the machine learning module (200) and to analyse the undersampled MRI data (300) using the machine learning module (204), wherein the machine learning module (204) is trained for identifying the predetermined feature in industrial samples (102) of the predefined sample type using a training set (302) comprising undersampled MRI data (300) of different training samples of the predefined sample type, wherein a fraction of the training samples comprises the predetermined feature and a fraction of the training samples does not comprise the predetermined feature.

## Description

The present invention relates to a method for automated non-invasive identification of a predetermined feature in a multitude of industrial samples of a predefined sample type, an inference module for analysing undersampled MRI data of an industrial sample, an MRI system comprising said inference module and a corresponding computer program product.

In modern industry, advanced automation technologies have resulted in higher production and productivity, wherein industrial processes have undergone severe changes in the past decades. In recent years, various industries have significantly increased the overall output, i.e. the number of processed industrial products per hour, resulting in high-throughput, i.e. fast processing and reduced processing times for each individual industrial product. At the same time, customers' expectations regarding product quality and/or the amount of official regulations have increased, so that high product qualities have to be accomplished despite the high productivity. Furthermore, several industries rely on accurate assessment of the features and quality of relevant base materials, e.g. the fertility status of an egg in the case of hatcheries or the expected productivity of seeds in the agriculture industry. Therefore, enabling accelerated analysis of industrial samples is highly relevant for various industrial fields, both for quality control and property prediction.

A promising approach to satisfy this demand and to allow for a high-throughput analysis of samples are NMR (nuclear magnetic resonance) based techniques and in particular the use of Magnetic Resonance Imaging (MRI). The use of MRI techniques for providing information about e.g. the interior of industrial products has recently moved into focus. Herein, several different concepts for analysing properties of industrial samples with imaging techniques are discussed in the prior art, e.g. in EP 3 483 619 A1, US 6,149,956 A, WO 02/059586 A2, US 10,338,015 B2 and US 2019/011383 A1. Due to its fundamental relevance for the application potential of MRI in industrial application, a lot of attention is given to the optimization of the sample throughput, which is i.a. strongly influenced by the potential scan speed and by the overall MRI processing rate.

Apart from reducing the number of samples, an approach for saving measurement time and for increasing the processing rate is to reduce the digitization rate and/or the number of experiments taken in the phase encoding dimension. This results in the acquisition of undersampled MRI data. Furthermore, shot repetition times for each scan and/or the number of scans taken in each sub-experiment can be reduced to further increase the processing rate. Using this approach, the overall time required for each sample can be reduced, so that an increase in throughput can be achieved, in particular when combined with techniques like parallel imaging.

However, the acquisition of undersampled MRI data in the so-called k-space, results in MRI images that have a reduced quality and in particular exhibit so-called aliasing artefacts, while shorter shot repetition times and lower number of scans can drastically reduce the signal-to-noise ratio and the overall quality of the obtainable MRI image. With MRI being an imaging technique, the assessment of sample quality and/or specific sample properties in the prior art relies heavily on the analysis of the obtained MRI image, in several cases by a process operator. As a consequence, the prior art is heavily focussed on improving the quality of MRI images obtained from undersampled MRI data using different approaches, including some concepts that rely on artificial intelligence and utilize e.g. artificial neural networks for removing artefacts from images. A big part of this development is driven by improvements that originate from the medical field and the application of MRI in the diagnosis of medical condition, wherein a medical professional is dependent on receiving high-quality and artefact-free MRI images. Related disclosure can e.g. be found in US 2021/224634 A1, US 11,170,543 B2 and US 2020/0305756 A1.

While these techniques for improving the image quality are in several cases effective at obtaining high quality MRI images from undersampled MRI data, the respective reconstruction of aliased MRI images based on undersampled MRI data typically requires powerful processors and large memory capacities, i.e. high performance computers. The amount of additional computational power required for enhanced image reconstruction adds to the initial resources needed for performing the complex multi-dimensional Fourier transform that is required for obtaining the basic MRI image from the undersampled MRI data in the first place. Depending on the available computational power, even the overall image reconstruction itself can become a factor that reduces the overall throughput.

It was the primary objective of the present invention to overcome or at least reduce the disadvantages of the prior art.

In particular, it was the objective of the present invention to provide for a powerful method for analysing industrial samples with a high throughput allowing for fast sample processing and broad applicability in in multiple industrial fields.

Herein, it was an objective of the present invention, that the respective method should be able to reduce the computational power required for the analysis and to decrease the processing times.

It was desired that the respective method should be applicable to a wide variety of industrial sample types and should allow for efficient simultaneous analysis of several samples and/or subsequent analysis with a high processing rate.

Furthermore, it was an additional objective of the present invention, that the respective method should yield particular reliable results, wherein it was desired that the susceptibility to human failure should be reduced as much as possible, wherein most desirably the requirement for trained process operators should be removed.

Likewise, it was a further objective of the present invention, that the respective method should be operable on a wide variety of different MRI systems.

It was a secondary objective of the present invention, to provide for a powerful MRI system and a corresponding inference module that are suitable for use in the respective method. Likewise, it was an additional objective of the present invention to provide for a computer program product that enables the respective method.

The inventors of the present invention have now found, that the above described objectives can surprisingly be achieved, if a method for automated non-invasive identification of a predetermined feature in a multitude of industrial samples of a predefined sample type is employed as defined in the claims. Herein, the inventors realized that the inherent uniformity and/or similarity between different industrial samples of the same type, e.g. a multitude of eggs or a multitude of seeds, opens a promising route for increasing the processing speed when using MRI and machine learning techniques. In particular, the inventors found that for industrial samples, unlike e.g. in the medical field, it is expendable to provide an MRI image as an output and to have the MRI image analysed. Instead it is in several cases sufficient to automatically identify a predetermined feature, i.e. its presence, absence or magnitude, wherein it is sufficient to provide the respective information as an output, rather than a processed MRI image. Insofar, the inventors developed an approach based on machine learning that synergistically utilizes this idea. For this, the inventors did not rely on techniques of increasing the MRI image quality but instead found that machine learning techniques can be directly applied to undersampled MRI data, in particular undersampled raw MRI data and MRI images that comprise aliasing artefacts, in order to identify, if a predetermined feature is present in the industrial samples or not. In fact, it was found that aliasing artefacts, that are known to result from processing undersampled raw MRI data and in several cases lead to a replication of features, like e.g. of the representation of a contaminant, can surprisingly be utilized for synergistically enhancing the identification capability of said feature by a machine learning module that is trained for this purpose. Thus, the inventors found that surprisingly advantage of the aliasing artefacts could be taken instead of trying to mitigate it, as the inventors observed that aliasing artefacts that result from processing of undersampled raw MRI data can be engineered to replicate features of interest. During development, the inventors found that this beneficial concept can be utilized even further, as the machine learning module, unlike humans, is not dependent on analysing fully processed MRI images. Instead, it was found, that the method can surprisingly be conducted to identify predetermined features directly from undersampled raw MRI data or processed MRI data that is not fully transformed into an MRI image, e.g. obtained after a two or three dimensional Fourier transform, thereby beneficially even reducing the amount of computational resources needed for the general image reconstruction. In other words, the inventors found that the high comparability of industrial samples of a given type and the strategy to limit the search to one or more specific predetermined features of interest in combination with a powerful machine learning technique and an adequate training, enables a direct inference based on undersampled imaging MRI data, thus allowing for very high throughput of industrial samples and a very reliable analysis of relevant key features with a low risk for human failure. Furthermore, the inventors found that the method can beneficially be made operable on a broad variety of industrially relevant MRI systems, if information about the hardware and the experimental parameters are included in the training set used for training the machine learning module. Overall, it was found to be a very beneficial side effect of the respective method, that less scans and shorter shot repetition times can be used in the MRI measurement as the machine learning module was found to be significantly less dependent on a signal-to-noise ratio that would be sufficient for reliable visual inspection.

The above described objectives are solved by the subject-matter of the present invention as defined in the claims. Hereinafter, the subject-matter of the invention is discussed in more detail, wherein preferred embodiments of the invention are disclosed. It is particularly preferred to combine two or more of the preferred embodiments to obtain an especially preferred embodiment. Correspondingly, especially preferred is a method according to the invention, that defines two or more features of preferred embodiments of the present invention. Also preferred are embodiments in which a feature of one embodiment that is to some extent designated as preferred is combined with one or more further features of other embodiments that are designated to some extent as preferred. Features of preferred MRI Systems, inference modules and computer program products result from features of preferred methods.

The present invention relates to a method for automated non-invasive identification of a predetermined feature in a multitude of industrial samples of a predefined sample type, the method comprising the steps of:
a) conveying an industrial sample of the predefined sample type into an MRI scanner,
b) recording in an MRI measurement for at least one slice or at least a partial volume of the industrial sample undersampled MRI data, comprising:
   - undersampled raw MRI data, comprising a multitude of time dependent signals for different phases, and/or
   - processed MRI data, obtained from processing undersampled raw MRI data, and
c) analysing the undersampled MRI data with an inference module for identifying a predetermined feature of the industrial sample using a machine learning module that is trained for identifying the predetermined feature in industrial samples of the predefined sample type from undersampled MRI data,
wherein the inference module comprises a memory storing the machine learning module and a processor for controlling the inference module, wherein the inference module is configured to provide the undersampled MRI data as an input to the machine learning module and to analyse the undersampled MRI data using the machine learning module, wherein the machine learning module is trained for identifying the predetermined feature in industrial samples of the predefined sample type using a training set comprising undersampled MRI data of different training samples of the predefined sample type, wherein a fraction of the training samples comprises the predetermined feature and a fraction of the training samples does not comprise the predetermined feature.

The method according to the invention is automated, i.e. operated by largely automatic equipment, thereby enabling high throughput and mostly removing the need for process operators. In other words, the method uses machinery and electronics to perform most tasks, so that it can be carried out by machines or computers without needing human control. In theory, the method can be only partly automated, e.g. by including additional manual process steps or by using a manual conveyor. However, operating mostly or entirely automated is preferred.

The term "non-invasive" is well-known to the skilled person and defines that the method is non-destructive and does not require incisions or the insertion of instruments into the sample, or the extraction of content from the sample.

The method of the present invention comprises the identification of a predetermined feature in a multitude of industrial samples of a predefined sample type.

The term "industrial samples" as used herein refers to samples coming from and/or being further processed in industrial facilities. In perfect agreement with the understanding of the skilled person, the term "industrial samples" does not comprise animals or humans. Therefore, the method according to the invention does not comprise identification of a predetermined feature in human beings and animals. Based on the needs of the respective industries and the excellent suitability of the method of the invention, preferred is a method according to the invention, wherein the industrial sample is designated for use in an industry that is selected from the group of agricultural industry, in particular farms and hatcheries, and food industry, in particular confectionery production or in the use of seeds and nuts.

The industrial samples that are employed in the method according to the invention belong to a predefined sample type. The term "predefined sample type" as used herein relates to a sample class or sample category which is defined prior to the method of the present invention and will in most cases depend on the industry. Thus, samples of a predefined type generally display similar properties so as to be comparable to one another. In other words, the predefined sample type is a grouping criterion to group a number of industrial samples according to their type, nature, origin or properties, including shape, volume, composition and colour. Particularly, a multitude of samples of a predefined type may or may not show the predetermined feature. Correspondingly, the method of the present invention is configured to identify whether a sample of the predefined type shows the predetermined feature. While this definition might appear unwieldy at first, there is no practical obstacle for the skilled person to define a sample type and to decide, if an industrial sample falls under the predefined sample type. For example, the predefined sample type may be "eggs", or more specifically "chicken eggs", or alternatively might be "pralines", or more specifically "pralines with cherry". In the vast majority of the cases, the predefined sample type will be whatever product is produced in the respective facilities. Insofar, the skilled person readily understands that the identification of a predetermined feature in a more generic predefined sample type, e.g. "seeds" instead of "grape seeds", requires more effort in training the machine learning module and typically will require a larger training set for obtaining the adequate machine learning module as disclosed below. While this is not a problem of the method according to the invention, defining a highly generic predefined sample type may reduce the overall value of the feature identification provided by the method, e.g. because a feature that is desired in grape seeds with respect to the expected yield might be totally uncorrelated to the yield in grain seeds. Thus, it is preferred for most cases to define the predefined sample type as narrow as reasonably possible and to adapt the training set accordingly.

The industrial samples of the predefined sample type can in principle be any industrially usable product or item that substantially exhibits similar features, i.e. falling under a suitable grouping criterion. However, the inventors have found that the method of the present invention can be employed to certain industrial samples in a very efficient way, in particular due to their chemical and structural composition and therefore their typical NMR properties as well as their typical size, that allows efficient measurement in comparably small MRI devices, that can be arranged more easily in typical factories. Specifically, a method according to the invention is preferred, wherein the predefined sample type is selected from the group consisting of polymers, animal products, plants, and products derived from these materials, preferably plastics, organic tissues, meat, fish meat, eggs, fruits, seeds and processed food and drinks, more preferably eggs, seeds, nuts and chocolate products.

The term "predetermined feature" as used herein refers to a feature that has been specified, set or established in advance of the method of the present invention, wherein "predetermined" and "predefined" are herein used interchangeably and attributed to the "feature" and "sample type", respectively, to allow for better distinguishing between the two aspects. The predetermined feature can be any characteristic, property, or attribute of the industrial sample, e.g. a biomarker. Particularly, the predetermined feature can e.g. be related to the industrial samples chemical composition, physical properties, and structural features but also e.g. size ratios of elements in the samples. Compared to most prior art techniques, that analyse the quality based on enhanced MRI images visually and derive conclusion from what is visible (either visually or with regular computer programs), the method of the present invention searches for a specific predetermined feature in the undersampled MRI data, by training the machine learning module for this respective feature.

The skilled person understands that the method according to the invention can be used to simultaneously identify two or more predetermined features on the same sample using the same undersampled MRI data, e.g. the presence of a cherry pit and/or a cherry stalk in a multitude of cherries. Insofar, the skilled person understands that the identification of a more generic predetermined feature, e.g. the identification of a more generic contaminant (including i.a. cherry pit, cherry stalk and worms) in the cherries, requires more effort in training the machine learning module and typically will require a larger training set for obtaining the adequate machine learning module. Thus, it is preferred for most cases to define the predetermined feature as narrow as reasonably possible. In comprehensive experiments the inventors identified several categories of predetermined features that can be identified very reliably with the method according to the invention. In particular, a method according to the invention is preferred, wherein the predetermined feature is selected from the group consisting of chemical composition, physical properties, in particular magnetic properties, and structural features, preferably structural features, more preferably anatomical features, biological features, morphological dimensions, sample structure, spatial distribution and size ratios of elements in the industrial sample and presence of impurities.

In the first step, the method of the present invention comprises the conveying of an industrial sample into an MRI scanner. The skilled person understands that the conveying can be performed either manually or, in a strongly preferred embodiment, automatically. The conveying can comprise a pre-sorting mechanism and/or a transfer mechanism. In a preferred embodiment, the conveying is controlled by a transport controller, particularly a transport controller communicating with a central controller, which also controls the MRI scanner. In particular, the conveying can be performed for multiple industrial samples at the same time, e.g. by using a suitable tray, wherein the method according to the invention preferably is conducted for all samples that are conveyed into the MRI scanner. In view of this, a method according to the invention is preferred, wherein a multitude of industrial samples is conveyed into the MRI scanner, sequentially and/or in parallel. Also preferred is a method according to the invention, wherein the multitude of industrial samples are arranged for conveying in a regular pattern, preferably in a matrix configuration, preferably on a holder or tray that is suitable for holding a multitude of industrial samples of the predefined sample type.

As discussed above, there is a need for high-throughput analysis that ensures the quality and/or properties of a large number of industrial samples per time. Insofar the skilled person understands, that while the method of the invention is defined for an individual industrial sample for reasons of clarity, the method according to the invention is not a laboratory scale experiment, that is typically conducted on just a single sample, but rather is designed for analysing a multitude of industrial samples, in particular vast numbers of industrial samples per time. Thus, a method according to the invention is preferred, wherein the method is applied for 2 or more, preferably 5 or more, more preferably 10 or more, most preferably 20 or more, in particular preferably, 1000 or more, even more preferably 5000 or more, industrial samples. Likewise, the term "an industrial sample" in the above definition could be replaced with "one, two or more industrial samples", preferably "two or more industrial samples"

Especially high throughput rates are typically obtained by capitalizing on the favorable potential of the method according to the invention to process several industrial samples at the same time and/or sequentially with a high rate, due to the beneficially low demand for computational resources and the ability to use highly undersampled MRI data. Correspondingly, it is preferred for basically all embodiments, to operate the method with a high sample throughput and/or processing rates. Therefore, a method according to the invention is preferred, wherein the method is sequentially applied to a multitude of industrial samples, that means that a multitude of samples are processed one after another, and/or wherein the method is simultaneously applied to a multitude of industrial samples, wherein preferably the method is sequentially applied several times simultaneously to a multitude of industrial samples. Correspondingly, a method according to the invention is preferred, wherein simultaneously for a multitude of industrial samples, preferably 3 or more, more preferably 5 or more, most preferably 20 or more, undersampled MRI data is recorded for at least one slice or at least a partial volume of each industrial sample. In particular, preferred is a method according to the invention, wherein the method is operated at a rate of 1000 or more industrial samples per hour, preferably 5000 or more industrial samples per hour. Additionally or alternatively, a method according to the invention is preferred, wherein the time for recording the undersampled MRI data for the industrial sample is in the range of 1 to 30 seconds, preferably in the range of 1 to 10 seconds, more preferably in the range of 1 to 5 seconds.

The general structure and function of MRI scanners and the concepts of MRI measurements, including typical MRI experiments, pulse sequences and techniques are well known to the skilled person and need not be described herein. MRI scanners that are suitable for the method according to the invention are commercially available from several suppliers and in most cases include the software and pulse programs required for recording MRI data or undersampled MRI data, respectively. More disclosure about the general concept of MRI is readily available in the prior art, e.g. from WO 2019/092265 A1. Yet, the inventors identified features of MRI scanners and measurement schemes that have proven to be very suitable for conducting the method of the present invention in an industrial environment.

With respect to the hardware, a method according to the invention is preferred, wherein the MRI scanner comprises a magnet that generates a static magnetic field, wherein the magnetic field preferably has a magnetic field strength in the range of 0.05 to 9.4 Tesla, more preferably in the range of 0.05 to 7 Tesla, most preferably in the range of 0.05 to 1 Tesla, wherein the MRI scanner typically comprises a measurement zone, that is typically located in the centre of the static magnetic field, wherein the industrial sample is in the measurement zone during the MRI measurement. The respective magnetic field strengths have proven to be a very efficient compromise between the price of the hardware, the respective maintenance costs and the workplace safety on the one hand and the achievable signal-to-noise ratios on the other hand. The surprisingly good performance of the method according to the invention when applied to undersampled MRI data with bad signal-to-noise ratios allows the usage of comparably low field strengths further contributing to the large degree of industrial applicability. Relevant for the vast majority of cases is a method according to the invention, wherein the MRI scanner comprises magnetic gradient coils in one, two or three dimensions, configured to induce a magnetic gradient in the static magnetic field, preferably in the measurement zone, and/or wherein the MRI scanner comprises one or more radio-frequency coils configured to apply radio-frequency magnetic fields, preferably in the measurement zone.

With respect to the MRI measurement, basically all typical MRI experiments and MRI pulse sequences can be used to record the undersampled MRI data, wherein the MRI experiment is adapted by the skilled person to the MRI scanner and the industrial sample based on his general knowledge. For example, the skilled person would not consider running a 31P-MRI-measurement on a sample that does not contain phosphorus. In particular, a method according to the invention is preferred, wherein the MRI measurement is a T1-weighted or T2-weighted, or rather T2*-weighted, MRI measurement, wherein the respective sequences that typically employ spin echo sequences, are known in the field. With respect to the possible nuclei, a method according to the invention is preferred, wherein the MRI measurement is a 1H-, 13C-, 23Na- or 31P-MRI-measurement, preferably a 1H- or 13C-MRI-Measurement, more preferably a 1H-MRI-Measurement.

It is neither possible nor expedient to generally define experimental parameters for the MRI experiment, as the relevant parameters depend strongly on the MRI scanner, the MRI experiment, the nuclei under study, the sample type, the industrial feature of interest, and several other parameters. However, adjusting the parameters of an MRI experiment is a routine task for the skilled person that is aware of how to measure and/or calibrate the parameters needed for adjustment and/or calibration of the MRI scanner. In practice, the different parameters are oftentimes optimized for obtaining the best signal-to-noise ratios, for example by incrementally adjusting the individual parameters. While it is possible to calibrate the experimental parameters on arbitrary reference samples, it is expedient to at least supplement this calibration with measurements on a reference sample of the predefined sample type. Correspondingly, especially preferred is a method according to the present invention, wherein the parameters of the MRI measurement, in particular pulse lengths, evolution times and repetition times, are obtained based on calibration experiments conducted on industrial samples of the predefined sample type.

In the method of the present invention undersampled MRI data is recorded for at least one slice or at least a partial volume of the industrial sample, wherein the slices can have a certain thickness and the volume, sometimes called "excitation volume", can be the entire volume of the industrial sample. The recording depends on the MRI measurement that is employed. In case of slices, the slice selectivity of the MRI measurement is achieved using known techniques, typically by using the gradient coils to provide for a gradient in the magnetic field strength that results in a gradient of resonance frequencies and therefore enables a spatial separation of nuclei in the sample. In the case of volumes, known techniques like 3D encoding techniques can be employed.

For several applications it can be very advantageous to record only exactly one slice of the industrial sample of the predefined sample type, e.g. for industrial samples with small dimensions or for overall time consuming MRI measurements, e.g. on samples with very long T1 times. Such a limitation can e.g. be employed in samples, for that a reliable prediction can be made about where the respective predetermined feature would be expected, e.g. for the cherry pit in cherries, that e.g. should be at least close to the center of the fruit, thereby removing the need to record undersampled MRI data for further slices. However, for other cases it can be expedient to record two or more slices of each industrial sample, e.g. for the identification of potentially randomly distributed predetermined features such as e.g. impurities or defects in industrial samples with a notable depth perpendicular to the slice. Therefore, a method according to the invention is preferred, wherein for the industrial sample, preferably for each industrial sample, the undersampled MRI data is recorded for 2 or more, preferably 3 or more, more preferably 5 or more, slices of the industrial sample, wherein the spacing between the samples is preferably chose to evenly distribute the slices though the volume of the industrial sample, wherein the slices are preferably recorded simultaneously. More specifically, preferred is a method according to the invention, wherein each slice has an average thickness in the range of 0,5 to 10 mm, preferably in the range of 0,75 to 5 mm, more preferably in the range of 1 to 2 mm. As an alternative, a method according to the invention is preferred, wherein undersampled MRI data is recorded for only one slice of the sample. Yet, for several applications a method according to the invention is preferred, wherein undersampled MRI data is recorded for at least a partial volume, preferably basically the entire volume, of the industrial sample.

For understanding the method of the present invention, understanding the distinction between "undersampled MRI data", "undersampled raw MRI data" and "processed MRI data obtained from processing undersampled raw MRI data" is of relevance as well as the difference to regular, non-undersampled MRI data. In agreement with the skilled persons understanding, MRI data comprises the raw MRI data that is obtained in an MRI measurement by sampling (or digitizing) the continuous time dependent NMR signal (e.g. FID or Echo) for a set of different experiments in the two- or three-dimensional experiment (also referred to a k-space) as well as any representations of undersampled raw MRI data that are derived thereof by processing, in particular by Fourier transforming. To obtain "undersampled MRI data", the sampling is performed on less points than intrinsically necessary to resolve the highest frequency contributing to the continuous time dependent NMR signal and/or to resolve the phase angle of the magnetization, respectively. For example, for the time dimension, the sampling rate for obtaining the discrete time dependent signal is below twice the highest frequency, i.e. the Nyquist rate, of the continuous time dependent NMR signal. A corresponding concept applies to the number of signals for different phases, that can also be described as time dependent signals with different spatial frequency components, wherein undersampling happens if the spacing between the phase increments is larger than the corresponding Nyquist interval. In agreement with the skilled persons understanding, the term "undersampled raw MRI data" comprises all minor modifications of undersampled raw MRI data that are merely arbitrary modifications like addition or subtraction of a fixed value or multiplication with a fixed value.

Apart from the undersampled raw MRI data, the term "undersampled MRI data" also comprises processed MRI data obtained from processing undersampled raw MRI data, wherein the processing can comprise in principle any data processing operation but in most cases will comprise a Fourier transform in one, two or three dimensions, eventually leading to an MRI image that comprises aliasing artefacts that are caused by the undersampling. Both the Nyquist rate and the Nyquist interval are well known to the skilled person and recording of undersampled MRI data poses no problem for the skilled person.

It is noted that, while the undersampled MRI data that is directly detected by the detectors will typically be undersampled raw MRI data, processing of undersampled raw MRI data and the resulting acquisition of processed MRI data is considered "recording" as would be the natural understanding of the skilled person, who would e.g. consider the acquisition of MRI images to be a recording of MRI images.

Undersampling allows for particularly fast MRI measurements and generally enables a high sample-throughput. This advantageous effect is even more pronounced, when the MRI data is undersampled in two dimensions or even three dimensions in case not only one slice is recorded and a three dimensional image shall be constructed using two phase-encoding dimensions. While undersampling in one dimension is typically considered "bad enough" in the prior art, undersampling in two or more dimensions is oftentimes considered not expedient in methods of the prior art. However, it is a large advantage of the present invention that the method according to the invention can readily be employed for reliable feature identification using MRI data that is undersampled in two or more dimensions and/or wherein the undersampling is particular severe by sampling with a large difference to the Nyquist rate and the Nyquist interval.

In view of the above explanations, a method according to the invention is preferred, wherein for obtaining undersampled MRI data, in the MRI measurement the sampling rate for the time dependent signal is below the Nyquist rate and/or the spacing between the phase increments is larger than the Nyquist interval, wherein preferably the sampling rate for the time dependent signal is set to be below the Nyquist rate and the spacing between the phase increments is set to be above the Nyquist interval. Preferred is a method according to the invention, wherein the sampling rate for the time dependent signal is 75 % or less, preferably 50 % or less, more preferably 25 % or less, of the Nyquist rate.

Preferred is likewise a method according to the invention, wherein the spacing between the phase increments is 200 % or more, preferably 300 % or more, more preferably 400 % or more, of the Nyquist interval. This corresponds to skipping lines in the k-space. Most preferably, the respective features are realized together with the corresponding degrees of preference.

Generally, the undersampling or the reduction of the sampling rate, respectively, can be achieved along any encoding dimension, that is along frequency, phase, or slice encoding.

As discussed above, undersampled MRI data may comprise either or both of "undersampled raw MRI data" and "processed MRI data obtained from processing undersampled raw MRI data". The latter refers to any type of processed MRI data obtained from the undersampled raw MRI data, particularly by performing a Fourier transformation in the phase-encoding and/or the timeencoding dimensions, preferably in both dimensions. It also refers to other linear or nonlinear encoding operations applied to the undersampled raw MRI data. The skilled person understands that it is expedient that no artificial generation of additional sampling points or any other method of increasing the data quality is performed on the processed MRI data, as such steps are not needed by the method of the invention. Due to being based on undersampled raw MRI data, the processed MRI data obtained therefrom by Fourier transforming along an undersampled dimension comprises at least one aliasing artefact. Within the skilled persons understanding, the aliasing artefact is an effect that causes different signals of the continuous-time NMR signal to become indistinguishable in the discrete time dependent signal. While these aliasing artefacts are often detrimental for prior art methods, they advantageously do not hinder the method according to the invention, at least not too much, and can even be beneficial for training the machine learning module as aliasing artefacts can result in replication of the predetermined feature in the sample, providing more features for the machine learning module to recognize. Correspondingly, if undersampled raw MRI data is obtained, a method according to the invention can be preferred, wherein the analysing comprises processing undersampled raw MRI data by Fourier transforming along at least one undersampled dimension, to obtain processed MRI data that comprises at least one aliasing artefact.

The highest processing rates and sample throughputs as well as the strongest reduction in required computational power is typically accessible if undersampled raw MRI data is directly analysed, wherein the exclusive analysis of undersampled raw MRI data is a preferred option in all embodiments. Overall, the capability of the machine learning module to identify the predetermined features in industrial samples in undersampled raw MRI data, in particular in undersampled k-space data, and the option of removing any Fourier transform or image processing steps, is among the most relevant advantages of the method according to the invention. Particularly preferred is therefore a method according to the invention, wherein the undersampled MRI data, comprises undersampled raw MRI data, wherein the undersampled MRI data, preferably consists of undersampled raw MRI data. As indicated above, preferred is also a method according to the invention, wherein the undersampled raw MRI data is undersampled in both the time dimension and the phase dimension. Correspondingly, a method according to the invention is preferred, wherein the machine learning module is trained for identifying the predetermined feature in industrial samples of the predefined sample type from undersampled raw MRI data.

It was found that training of machine learning modules for identifying the predetermined feature in industrial samples of the predefined sample type from undersampled raw MRI data can be more challenging than providing more processed data, thus e.g. requiring larger training sets. For several applications it is therefore preferred to use processed MRI data. Thus, a method according to the invention is preferred, wherein the undersampled MRI data, comprises processed MRI data, wherein the undersampled MRI data, preferably consists of processed MRI data.

Herein, a very preferred route uses highly processed MRI data and even MRI images with aliasing artefacts, in particular as several MRI scanners are optimized for returning MRI images as an output so that the respective data is sometimes more readily available. In view of this, a method according to the invention is preferred, wherein the processed MRI data is obtained from processing undersampled raw MRI data using a one-, two- or three-dimensional, preferably two-dimensional, Fourier Transform, in particular along the frequency-encoding dimension and/or the phase-encoding dimension, and/or wherein the processed MRI data is the one-, two- or three-dimensional, preferably two-dimensional, Fourier transform of undersampled raw MRI data, in particular along the frequency-encoding dimension and/or the phase-encoding dimension. Likewise preferred is a method according to the invention, wherein the processed MRI data is obtained from processing undersampled raw MRI data by Fourier transforming along at least one undersampled dimension, to obtain processed MRI data that comprises at least one aliasing artefact, wherein the machine learning module is trained for identifying the predetermined feature in industrial samples of the predefined sample type from processed MRI data that comprises at least one aliasing artefact. Particularly preferred is a method according to the invention, wherein the processed MRI data is obtained as an MRI image by Fourier transforming or otherwise linearly or nonlinearly encoding, preferably by Fourier transforming, the undersampled MRI raw data along an undersampled frequency-encoding dimension and an undersampled phase-encoding dimension, wherein the MRI image comprises at least one aliasing artefact, wherein the machine learning module is trained for identifying the predetermined feature in industrial samples of the predefined sample type from MRI images that comprise at least one aliasing artefact.

However, a very efficient method according to the invention is obtained as a good compromise between efficiency of the analysis and required computational power if a method according to the invention is employed, wherein the processed MRI data is obtained from processing undersampled raw MRI data by Fourier transforming along exactly one undersampled dimension, to obtain processed MRI data that comprises at least one aliasing artefact. The use of undersampled raw MRI data and/or processed MRI data with a low degree of processing rather than full MRI images was found to be a very advantageous embodiment.

In the third step, the method of the present invention comprises analysing the undersampled MRI data with an inference module for identifying the predetermined feature of the industrial sample. The skilled person understands that the result of the method according to the invention is a feedback of the inference module and thus the information if the predetermined feature is present in the industrial sample or present in a specific magnitude, respectively. Therefore, the method according to the invention for example returns a comparably simple answer in the form of "yes", "no" or "partially", thus contributing to the advantageous fast processing rates. In view of the method according to invention it is neither necessary nor expedient that the inference module also returns MRI data, in particular processed MRI data, as this would in most cases only increase the amount of required computational power and the need for additional hardware, e.g. in the form of displays. Therefore, a method according to the invention is preferred, wherein the inference module does not comprise means for outputting visualized output of processed MRI data, in particular MRI images. Likewise, a method according to the invention is preferred, wherein the inference module is not configured to improve the resolution of an MRI image and/or to reconstruct an MRI image and/or to remove artefacts from an MRI image and/or to improve the quality of an MRI image in another way, wherein most preferably none of these process steps are performed in the method according to the invention. Especially preferred is a method according to the invention, wherein no processed MRI data is provided as an output of the inference module.

Preferred in the vast majority of cases is a method according to the invention wherein the inference module is configured to provide the result of the analysing in the form of a classification and/or an assessment parameter, and/or wherein in step c) the inference module provides the result of the analysing, preferably in the form of a classification and or an assessment parameter.

In view of the above, it is highly preferred to classify the industrial samples depending on the identification of the feature. This means that two or more classes are provided that are based on a grouping criterion, wherein the industrial samples are assigned to one or more of these classes based on the identification of the predetermined feature. For example, if the predetermined feature is the amount of water in glass bottles, the classes could e.g. be i) less than 95 %, ii) between 95 % and 98 % and iii) more than 98 %, wherein e.g. only samples of class ii) would be further processed by sealing the bottles as those are neither too full nor too empty. In a very efficient embodiment this classification can be done automatically by the inference module. Thus, preferred is a method according to the invention, further comprising the step of:
d) classifying the industrial sample based on the result of the analysis, wherein the classification is preferably conducted by the inference module, wherein the inference module is preferably configured to classify industrial samples of the predefined type based on the result of the identification of the predetermined feature, wherein the multitude of industrial samples is most preferably conveyed out of the MRI scanner and sorted based on the classification.

Taking into account the above disclosure, it is understood that a large benefit of the present invention consists of the high usability and reliability of the fast identification of predetermined features and/or of the fast classification of the industrial samples that makes the method of the present invention particularly suited to control subsequent processing steps, like e.g. the transfer of eggs to a hatchery or the packaging of products that are free of impurities, based on the output. Therefore, preferred is a method according to the invention, wherein the method identifies the presence, absence or magnitude of the predetermined feature and/or wherein subsequent processing steps of the industrial sample are controlled in dependence of the presence, absence or magnitude of the predetermined feature. Furthermore, preferred is a method according to the invention, wherein the industrial sample is only provided for further processing if the result of the analysing, preferably the presence, absence or magnitude of the predetermined feature, meets a predetermined quality criterion, wherein the industrial sample is preferably disposed and/or repurposed if the predetermined quality criterion is not met.

The inference module used in the method of the present invention is used for analysing the undersampled MRI data for identifying a predetermined feature of the industrial sample. The inference module itself is a physical device and can be a typical data processing device, e.g. a computer. In agreement with this, the inference module comprises a memory, i.e. a computer-readable storage, for storing e.g. data or software as well as a processor, i.e. a digital circuit which is able to perform operations on an external data source, for controlling the inference module. As the hardware infrastructure of the inference module is not critical for the method of the present invention, it is expedient to define the presence of only the basic components of an electronic data processing system, i.e. the memory and the processor, wherein for both of these components typical elements can be used that are commercially available, wherein the inference module can also comprise other components of typical data processing systems, e.g. power supply, mouse, keyboard, displays, network connectors etc. The inference module can e.g. be a part of a central controller that also controls the conveyor and/or the MRI scanner.

The processor of the inference module is used for controlling the inference module, wherein the inference module is configured to provide the undersampled MRI data as an input to the machine learning module and to analyse the undersampled MRI data using the machine learning module. The skilled person understands that this configuration is established in the typical way, e.g. by using software that can be programmed by the skilled person himself or provided by typical specialized programming companies. Thus, the inference module typically comprises computer executable code, that can be stored in the memory, which comprise machine executable instructions or a program which causes a processor and the inference module to perform their respective tasks in the method of the present invention. Suitable designs for assemblies from processors and memory are e.g. disclosed in EP 3 704 666 B1.

The core component of the inference module is the machine learning module that is stored in the inference module's memory, and therefore usable by the inference module for analysing the undersampled MRI data by providing the undersampled MRI data to the machine learning module. The machine learning module processes the input and identifies the predetermined feature, in particular the presence, magnitude or absence of the predetermined feature, wherein the output of the machine learning module is processed by the inference module and the processor, respectively, wherein the output of the machine learning module can e.g. be stored in a memory, provided to a user via an interface or provided as an input into subsequent processing devices, e.g. sorters.

In recent years, employing machine learning techniques or so-called artificial intelligence has become a promising approach for solving different technical problems in several industrial fields. Today, machine learning, its core concepts and its implementation are well-known concepts and several companies offer commercial solutions for implementing machine learning. While some skilled persons that have their focus in the field of MRI and/or in the field of quality control would potentially have to study some literature before setting up some of the more complex machine learning solutions entirely on their own, this skill is not necessarily required for putting the method of the invention into practice. Suitable machine learning algorithms and codes are available from the prior art and from commercial service providers if required. In practice, in most industries the implementation of the machine learning aspects of the present invention will probably be provided by skilled persons in the field of machine learning that have no problems in providing a suitable machine learning module in view of the disclosure of the invention, wherein these skilled persons can e.g. be part of a team of employees or personal of external IT-service providers. Insofar, the machine learning module is similar to the MRI scanner in that it oftentimes will be provided by specialized companies.

The inference module uses the machine learning module for analysing the undersampled MRI data. In agreement with the understanding of the skilled person, the machine learning module is the entity, e.g. a program, that is actually analysing the undersampled MRI data and can provide for the desired identification of the predetermined feature, typically without task-specific programming. For this, the machine learning module typically comprises both data and a procedure for using the data to make a prediction, sometimes called prediction algorithm. The machine learning module itself does not necessarily need to be able to further "learn" or "train" itself, but can exist, e.g. in the form of a program comprising the machine learning module, and continuously serve its task in the same way.

In agreement with the skilled persons understanding, machine learning modules can be obtained by running (or fitting) a machine learning algorithm, sometimes called learning rule or learning algorithm, on a data set, that is oftentimes called training set. Thus, by running a machine learning algorithm on a training set of data a machine learning module can be obtained or an existing machine learning module can be modified. Thus, by using the machine learning algorithm on the training set the machine learning module is trained for its specific task. In the framework of the present invention and in agreement with the established wording, the machine learning module is defined to be trained, wherein the training is specified in particular by defining the training set. An exemplary disclosure for the use of machine learning in the field of MRI can be found in EP 3 704 666 B1, that provides additional information for further understanding of the technological background.

In practice, the choice of the machine learning algorithm used for obtaining the machine learning module will in most cases depend on the industrial samples and, oftentimes more importantly, the predetermined feature that needs to be identified. Yet, the inventors were able to identify suitable machine learning algorithms that can be used to generate the machine learning module, that is correspondingly based on the respective machine learning algorithm. In particular, a method according to the invention is preferred, wherein the machine learning module is based on a machine learning algorithm, wherein the machine learning algorithm is selected from the group consisting of regression algorithms, linear classifiers, instance-based algorithms, regularization algorithms, decision tree algorithms, bayesian algorithms, clustering algorithms, association rule learning algorithms, artificial neural network algorithms, deep learning algorithms, dimensionality reduction algorithms and ensemble algorithms, preferably linear classifiers, artificial neural network algorithms and deep learning algorithms. Correspondingly, a method according to the invention is preferred, wherein the machine learning module is obtained by applying a machine learning algorithm on the training set, wherein the machine learning algorithm is selected from the group consisting of regression algorithms, linear classifiers, instance-based algorithms, regularization algorithms, decision tree algorithms, bayesian algorithms, clustering algorithms, association rule learning algorithms, artificial neural network algorithms, deep learning algorithms, dimensionality reduction algorithms and ensemble algorithms, preferably linear classifiers, artificial neural network algorithms and deep learning algorithms. Herein, the machine learning algorithms themselves are known in the prior art and can be employed by the skilled person in view of the industrial sample and the predetermined feature.

Based on this, the inventors identified specific types of machine learning modules that have proven to provide excellent results in the identification of predetermined features in industrial samples and that are therefore explicitly preferred for all embodiments. Therefore, a method according to the invention is especially preferred, wherein the machine learning module is a deep learning network or an artificial neural network, preferably a deep learning network. Herein, these types of machine learning modules are known to the skilled person and e.g. described in more detail in EP 3 704 666 B1.

Due to the large amounts of undersampled MRI data that are typically recorded during industrial scale use of the method of the present invention, it is highly recommended to provide for a mechanism for continued training of the machine learning module, to continuously enhance the performance and accuracy of the identification. Thus, a method according to the invention is especially preferred, wherein the inference module comprises a machine learning algorithm for generating a machine learning module or training a machine learning module based on a set of undersampled raw MRI data collected by using the method, wherein the machine learning algorithm is selected from the group consisting of regression algorithms, linear classifiers, instance-based algorithms, regularization algorithms, decision tree algorithms, bayesian algorithms, clustering algorithms, association rule learning algorithms, artificial neural network algorithms, deep learning algorithms, dimensionality reduction algorithms and ensemble algorithms, preferably linear classifiers, artificial neural network algorithms and deep learning algorithms.

While the skilled person chooses the machine learning algorithm based on his general knowledge and his specific needs, it is the training of the machine learning module and therefore the training set that connects the machine learning modules of the present invention with each other. According to the invention, the machine learning module is trained for identifying the predetermined feature in industrial samples of the predefined sample type using a training set comprising undersampled MRI data recorded for different training samples of the predefined sample type, wherein a fraction of the training samples comprises the predetermined feature and a fraction of the training samples does not comprise the predetermined feature. Therefore, the training of the machine learning module is a supervised training, i.e. a training based on input data with known results. In this training process predictions are made, which are manually or automatically corrected when turning out wrong. The desired level of accuracy is achieved through repetitions of the aforementioned process.

The training samples for the training of the machine learning module for identifying the predetermined feature in industrial samples of the predefined sample type are therefore represented by industrial samples of the same predefined sample type, i.e. egg training samples for identifying predetermined features in eggs, with a known result, i.e. whether the eggs exhibit the predetermined feature or not. In other words, the presence or absence of the predetermined feature in the training samples is known prior to the training of the machine learning module. It is known that the typical accuracy of the prediction i.a. depends on the quantity and quality of the training samples.

For some machine learning based techniques, e.g. for improvement of image quality or for the removal of artefacts like disclosed in e.g. EP 3 704 666 B1, methods are suggested for generating an artificial training set. However, the inventors found that this is hardly possible and, more importantly, not expedient for the method of the present invention. Due to the fact that the method according to the invention is employed in high throughput industries and directed at the identification of features that are relevant for these industries, potential samples that exhibit the predetermined feature (or not, respectively) are typically available in sufficient numbers, at least after a few days of operation. In most cases, suitable samples can also be generated artificially, e.g. by intentionally not removing the cherry pits from a set of training cherries. The required training data is readily obtained by recording undersampled MRI data in an MRI measurement for at least one slice or at least a partial volume of the training sample, as it is described for the method of the invention above. Insofar, as typically only a few slices or a partial volume of the industrial sample will be analysed, it is easily possible to multiply the amount of available training data by changing the position and/or orientation of the industrial sample in the MRI scanner and conducting a new MRI measurement. Overall, the use of training sets that are obtained on real samples with real predetermined features is preferred due to typically yielding the most potent machine learning modules. Herein, the respective training set of undersampled training MRI data can also be readily obtained by the skilled person by recording undersampled MRI data on exemplary samples from his industrial process, wherein preferably the same MRI scanner can be used, that is employed in the method according to the invention. In view of the above, a method according to the invention is preferred, wherein the training set comprises undersampled MRI data of different training samples of the predefined sample type that was recorded with the same type of MRI scanner used in the method, wherein preferably similar, more preferably basically identical, experimental parameters were employed for recording the undersampled MRI data of the training samples as are used in the method.

As an alternative to the above generation of a training set, the skilled person can employ a training set that was externally provided, e.g. recorded on a reference facility, wherein it is preferably to include information about the experimental parameters used in the respective MRI measurements and/or to vary the experimental parameters of the MRI measurements during acquisition of the training set in order to increase the adaptability of the training set and/or the trained machine learning module to different MRI scanners and/or different experimental parameters. Thus, a method according to the invention is preferred, wherein the machine learning module is trained for identifying the predetermined feature in industrial samples of the predefined sample type using the training set, wherein each undersampled MRI data of different training samples is linked with information about the MRI Scanner and/or the experimental parameters of the MRI measurement used to obtain the undersampled MRI data, so that the machine learning module is trained for identifying the predetermined feature in industrial samples of the predefined sample in a multitude of different MRI scanners and/or under different experimental conditions, wherein the inference module is configured to provide information about the MRI scanner and/or the experimental parameters of the MRI measurement of the method as an input to the machine learning module, wherein the experimental parameters are preferably selected from the group comprising pulse lengths, pulse sequence, evolution times, repetitions times, sampling rate, phase increments, temperature and number of scans.

The inventors found that the best training results for the machine learning module are typically obtained, if the amount of training samples that comprises the predetermined feature is comparably large. Specifically, a method according to the invention is preferred, wherein the fraction of the training samples that comprises the predetermined feature is in the range of 20 to 80 %, preferably in the range of 30 to 70 %, more preferably in the range of 40 to 60 %, most preferably in the range of 45 to 55 %. Advantageously, if two or more predetermined features shall be identified, the machine learning module can in most cases be trained with a training set in that the amount of training samples that do not exhibit any of the predetermined features can be significantly reduced, as training samples that only exhibit feature A can be used as a negative example for feature B.

Only for the sake of completeness it is noted that the skilled person understands that a strong lack of coherence between the training samples and the industrial samples employed in the method according to the invention can reduce the efficiency of the method, in particular when combined with a comparably generic definition of the sample type. For example, if the skilled person intends to use the method of the present invention on chicken eggs, he is aware that the accuracy and efficiency of the method according to the invention may be reduced, if the employed training set for eggs comprises 90 % undersampled MRI data on ostrich eggs.

Likewise, the skilled person is aware that the above definition of the method according to the invention and the training of the machine learning module implies that the training set comprises undersampled MRI data in the same format as the analysed undersampled MRI data. The skilled person that intends to analyse undersampled k-space data for chicken eggs would not consider a machine learning module that was trained on undersampled 3D-MRI-lmages of chicken eggs.

The inventors of the present invention applied the method of the present invention to three exemplary industrial applications. For all of these cases, the method according to the invention was found to be particularly effective in determining predetermined features of the industrial samples in a fast and reliable way. For example, the hatchability of incubated eggs was predicted based on the identification of the relative sizes of the egg compartments. Likewise, seeds were classified for expected productivity based on the identification of specific features that are associated with increased productivity. Furthermore, food samples, in particular chocolate pralines were analysed for the presence of contaminants, in particular cherry pits.

In view of the above, a method according to the invention is preferred, wherein the predefined sample type and the industrial sample is an egg, preferably a bird egg, more preferably a chicken egg, wherein the predetermined feature is selected from the group consisting of the shape of the egg, the volume of the inner egg elements, texture of the inner egg elements, chemical composition of the inner egg elements, relative position of the inner egg elements, absolute position of the inner egg elements, magnetic properties of the inner egg elements and features derived from this features, preferably the volume of the yolk, the volume of the white, the texture of the yolk, the texture of the white, the size ratio of the inner egg elements, the position of the blastoderm, and inner egg elements relative angular position in the egg. Correspondingly, a method according to the invention is preferred, wherein the hatchability of the egg is predicted based on the presence, absence or magnitude of the predetermined feature, preferably based on the volume of the egg white and/or the volume of the egg yolk and/or the ratio of these volumes and/or the position of the blastoderm.

Likewise, a method according to the invention is preferred, wherein the predefined sample type and the industrial sample is a seed, wherein the predetermined feature is selected from the group consisting of the seed filling, seed texture, seed volume, seed shape, the seed's water content and the seed's lipid content. Preferred is insofar a method according to the invention, wherein the quality and/or productivity of seeds is predicted based on the presence, absence or magnitude of the predetermined feature.

Moreover, a method according to the invention is preferred, wherein the predefined sample type and the industrial sample is food, wherein the predetermined feature is selected from the group consisting of the foods inner structure, in particular the texture, the food ingredients and/or contaminants' spatial distribution, and the position and size of contaminants in the food. Preferred is also a method according to the invention, wherein the compliance with product standards is evaluated based on the presence, absence or magnitude of the predetermined feature.

It is clear to the skilled person, that the invention also relates to an inference module for analysing undersampled MRI data of an industrial sample of a predefined sample type, using a machine learning module, preferably in a method according to the invention, wherein the inference module comprises a memory storing the machine learning module and a processor for controlling the inference module, wherein the inference module is configured to provide undersampled MRI data as an input to the machine learning module and to analyse the undersampled MRI data using the machine learning module, wherein the machine learning module is trained for identifying the predetermined feature in industrial samples of the predefined sample type using a training set comprising undersampled MRI data of different training samples of the predefined sample type, wherein a fraction of the training samples comprises the predetermined feature and a fraction of the training samples does not comprise the predetermined feature. The inference module can be connected with a suitable MRI, e.g. a regular industrial MRI scanner, to enable the method of the invention.

In view of this, it is clear to the skilled person that the present invention likewise relates to an MRI system for conducting the method according to the invention, comprising:
a) an MRI scanner for obtaining undersampled MRI data of industrial samples of a predefined sample type,
b) a conveyor for conveying a multitude of industrial samples into the MRI scanner, and
c) an inference module according to the invention, that is connected to the MRI scanner.

The invention also relates to a computer program product comprising instructions which, when the program is executed by a computer, preferably by an inference module according to the invention, cause the computer to carry out the step c), preferably the steps b) and c), more preferably all steps, of the method according to the invention.

Also disclosed herein are a computer-readable data carrier having stored thereon the computer program product according to the invention as well as a data carrier signal carrying the computer program product according to the invention.

Hereinafter, the invention is described in more detail, wherein preferred embodiments of the invention are disclosed with respect to the figures. The figures show:
- Fig. 1: a schematic visualization of the steps of the method according to the invention;
- Fig. 2: a schematic visualization of the structure of an inference module according to the invention;
- Fig. 3: a schematic visualization of the principle of the method according to the invention; and
- Fig. 4: a schematic representation of an exemplary MRI system according to the invention.

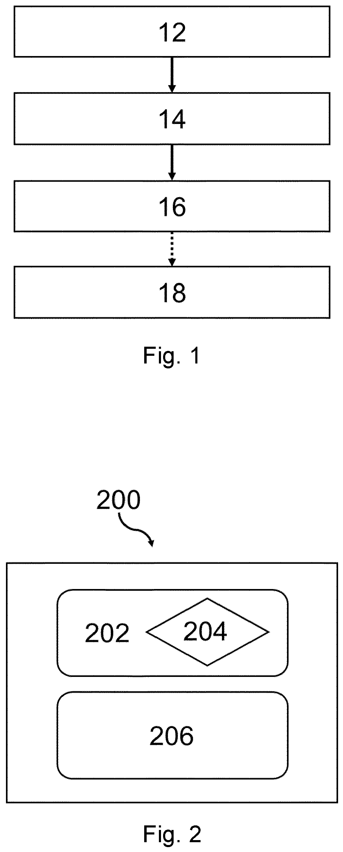

Fig. 1 provides a schematic visualization of an exemplary method according to the invention for automated non-invasive identification of a predetermined feature in a multitude of industrial samples 102 of a predefined sample type to facilitate the understanding of the present invention. In a first step 12 of the method, at least one industrial sample 102 is conveyed into an MRI scanner 106. In a second step 14 an MRI measurement is conducted on the industrial sample 102 to record undersampled MRI data 300 for at least one slice or at least a partial volume of the industrial sample 102. In a third step 16, the undersampled MRI data 300 acquired in the second step 14 is analysed with an inference module 200. The inference module 200 comprises a memory 202 with a machine learning module 204 stored thereon and a processor 206, which is configured to control the inference module 200. The analysing procedure within the inference module 200 in the third step 16 is performed using the machine learning module 204, wherein the undersampled MRI data 300 recorded in the second step 14 serves as an input to the inference module 200 and the machine learning module 204, respectively.

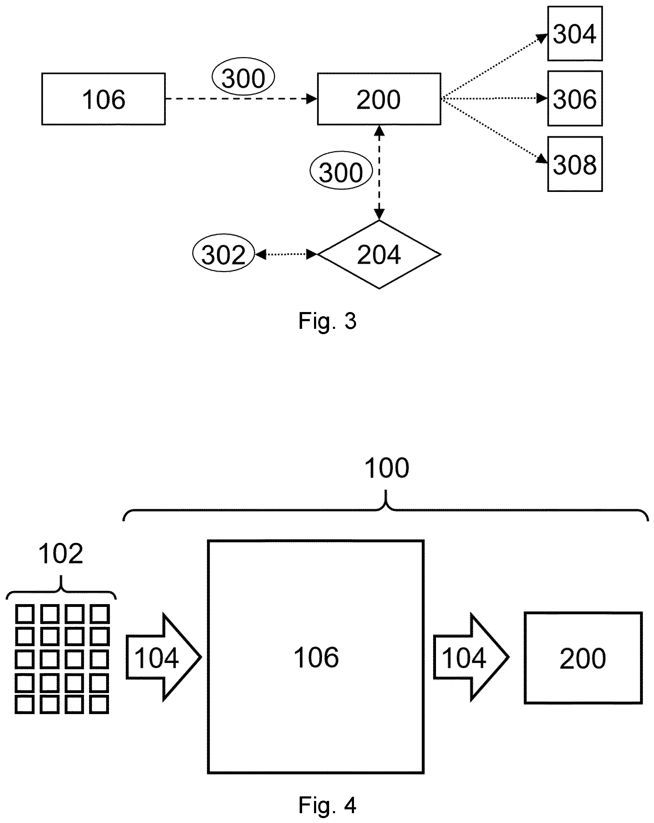

A schematic visualization of the structure of an inference module 200 that is suitable for use in the method according to the invention, in particular for the method shown in fig. 1 and 3 as well as for the MRI system 100 of fig. 4, is provided in fig. 2. Herein, the machine learning module 204 is trained for identifying a predetermined feature in industrial samples 102 of the predefined sample type. The training is made using a suitable machine learning algorithm and a training set 302 comprising undersampled MRI data 300 of different training samples of the predefined sample type in order to allow for supervised training. The training set 302 for example consists of undersampled MRI data 300 of training samples of the predefined sample type with and without the predetermined feature. In other words, a fraction of the training samples comprises the predetermined feature and a fraction of the training samples does not comprise the predetermined feature. For example, the fraction of the training samples in the training set 302 that comprises the predetermined feature is in the range of 45 to 55 %. Based on this training of the machine learning module 204, the method is able to identify the presence, absence or magnitude of the predetermined feature.

In the preferred embodiment depicted in fig. 2, the inference module 200 does not comprise means for providing visualized output of processed MRI data like MRI images, for example in the form of a display. Correspondingly, the inference module 200 is not configured to improve the quality of MRI images in any way but rather is configured to provide the result of the identification and potentially of the classification, for example to subsequent processing devices like e.g. sorting devices.

In an especially preferred embodiment of the inference module 200 according to fig. 2, that is a preferred option for all embodiments of the method or the MRI System 100, the machine learning module 204 is based on an artificial neural network algorithm or deep learning algorithm. Accordingly, the machine learning module is an artificial neural network or a deep learning module, wherein preferably the inference module 200 also comprises the machine learning algorithm for further training the machine learning module based on the undersampled MRI data 300 recorded in the method of the invention.

Fig. 1 depicts a preferred embodiment of the method according to the invention that comprises a fourth step 18. In this step, the industrial sample 102 is also classified by the inference module 200. The classification is based on the result of the analysis performed in the third step 16, wherein the result comprises information on the presence and/or absence and/or magnitude of the predetermined feature in the industrial samples 102 of the predefined sample type. In a preferred embodiment, the classification performed in the fourth step 18 is used for sorting the industrial samples 102 based on the result, preferably directly upon conveying the industrial samples 102 out of the MRI scanner 106. For example, the industrial sample 102 can be classified in three classes 304, 306 and 308, as is schematically visualized for an exemplary method according to the invention in fig. 3. In this example, the first class 304, the second class 306 and the third class 308 correspond to the classification as "suitable" for further processing, "not suitable" for further processing, and "unclear/repeat", wherein only those industrial samples 102 are further processed that are classified into the first class 304, while those industrial samples 102 that are considered "not suitable" in the second class 306 are conveyed out of the MRI scanner 106 for disposal or repurposing of the sample for another use. Finally, those industrial samples 102 that were classified as "unclear/repeat" in the third class 308, for example due to insufficient acquisition of undersampled MRI data 300 in the second step 14, are reintroduced into the MRI scanner 106, wherein the method is repeated to allow for classification in the first class 304 or second class 306. Insofar, fig. 3 provides for a highly schematic visualization of the use of the machine learning module 204 for a subsequent classification by the inference module 200 as described above. Undersampled MRI data 300 is provided to the inference module 200 by the MRI scanner 106 and analysed using the machine learning module 204, as indicated by the dashed double arrow. The machine learning module 204 was trained using a training set 302 comprising undersampled MRI training data as indicated by the dotted double arrow.

An exemplary MRI System 100 according to the invention that comprises an inference module 200 according to the invention and is thus suitable for conducting the method of the present invention is schematically depicted in fig. 4. The MRI scanner 106 comprises e.g. a magnet that generates a static magnetic field, wherein the magnetic field e.g. has a magnetic field strength in the range of 0.05 to 1 Tesla. The MRI scanner 106 comprises a measurement zone that is located in the center of the static magnetic field, so that the industrial sample 102 is in said measurement zone during the MRI measurement. The MRI scanner 106 typically comprises magnetic gradient coils in three dimensions, configured to induce a magnetic gradient in the static magnetic field, and radio-frequency coils, configured to apply radio-frequency pulses to the industrial samples 102, wherein the latter are typically arranged next to the measurement zone. For example, the magnetic gradient coils and the radio-frequency coils can be controlled by a gradient controller and a radio-frequency controller, respectively. A radio-frequency detector can be used to detect the signal, so that undersampled raw MRI data is recorded that can be provided to the inference module 200.

As is shown schematically in fig. 4, several industrial samples 102 are conveyed into the MRI scanner 106 by the conveying device 104 in the direction indicated by the arrow. The conveying device 104 is e.g. controlled by a transport controller, which is e.g. governed by a central controller, that also supervises the other controllers within the MRI scanner 106, e.g. through data channels or wireless communication.

In the example of fig. 4, the industrial samples 102 are arranged for conveying in five columns parallel, i.e. in a regular pattern that is defined by a suitable holder (not shown). Therefore, the method according to the invention is applied for multiple, i.e. 20 industrial samples 102, simultaneously. After undersampled MRI data 300 was recorded for said holder of industrial samples 102, the holder is conveyed out of the measurement zone and the next holder is arranged in the MRI scanner 106 for the MRI measurement. Thus, the method is sequentially applied several times simultaneously to a multitude of industrial samples 102. For example, the undersampled MRI data 300 is recorded for just one slice through each industrial sample 102, wherein the column arrangement of the industrial samples 102 allows for very efficient measurements and easy adjustment of the magnetic gradient coils 115. In an alternative, e.g. the entire volume of each industrial sample 102 can be recorded, e.g. by 3D acquisition techniques.

For example, the sampling rate for the time dependent signal in the MRI measurement can be set to just 25% of the Nyquist rate. Additionally, the spacing between the phase increments is 400% of the Nyquist interval, thereby skipping lines in the k-space. Other experimental parameters of the MRI measurement, such as pulse lengths or repetition times, can be obtained based on typically calibration experiments conducted on industrial samples 102 of the predefined sample type, e.g. on training samples contained in the training set 302.

Preferably, the method is optimized to the time for recording the undersampled MRI data 300 for the industrial samples 102 of one holder is below 5 s, wherein it is possible to operate the method at a rate of at least 5000 industrial samples 102 per hour or more.

In an example of the above method that is optimized for high throughput and low demand for computational power, the undersampled MRI data 300 consists of undersampled raw MRI data, e.g. undersampled k-space data.

In an alternative example of the above method that is optimized to provide for the highest verifiability through a process operator, the undersampled MRI data 300 consists of undersampled MRI images that comprise at least one aliasing artefact.

In a second alternative example of the above method that is considered an advantageous compromise between the two other methods, the undersampled MRI data 300 consists of processed MRI data that is obtained from Fourier transforming undersampled raw MRI data along one undersampled dimension.

The method according to the invention was employed for three different applications from different industries, wherein the method was found to be highly suitable for identifying predetermined features in a broad variety industrial samples 102 of a predefined sample type and to classify the industrial samples 102 if required.

In a first demonstration, eggs 102 were studied using the method of the present invention, wherein both the volume of the yolk and the volume of the white were determined on a central slice through the egg. Subsequently, the hatchability of the egg was predicted based on the magnitude of the ratio of the volumes of the white and yolk. The inventors found that an increased chance of hatchability of 83 to 88 % was found in incubated chicken eggs, if the ratio of sizes of the egg yolk divided by the egg white, estimated based on a slice going through the centre of the egg, was in the range of 0.44 to 0.50. Correspondingly, in the method of the invention, the predetermined feature was set to be that said ratio is in the range of 0.44 to 0.50. Using this approach, large numbers of chicken eggs could be analysed, wherein those that exhibited the feature were classified as "high hatchability" and labelled correspondingly. For the respective analysis, processed MRI data, obtained by a two-dimensional Fourier Transform of undersampled raw MRI data along both the frequency-encoding dimension and the phase-encoding dimension was used, wherein the undersampled raw MRI data was comprising aliasing artefacts. Other types of undersampled MRI data 300 could be used instead. The training set 302 was readily obtained by using undersampled MRI data 300 recorded on incubated eggs with the same MRI scanner 106 used before, wherein the sizes of the compartments in the training samples and their ratio were analysed manually using a typical software for image analysis, to allow for supervised learning.

In a second demonstration, chocolate pralines with a central cherry were analysed for a potentially incomplete removal of the cherry pit, in order to classify the pralines for their compliance with product standards. To obtain for particular high measurement rates, undersampled raw MRI data, which is undersampled in both the time dimension and in the phase dimension, can be used, wherein a particular high efficiency is obtained, as the predetermined feature and its potential location in the praline is quite well defined. However, other types of undersampled MRI data could be used instead. The training set 302 was readily obtained by using undersampled MRI data 300 recorded on chocolate pralines with a central cherry in that the cherry pit was not completely removed intentionally.

In a third demonstration, the method was employed to identify several features, like volume and water content, in apple seeds, as these features are often linked to the expected productivity. Herein, undersampled MRI data 300 was used that was obtained by Fourier transforming undersampled raw MRI data along one undersampled dimension. However, other types of undersampled MRI data could be used instead. The training set 302 was readily obtained by using undersampled MRI data 300 recorded on apple seeds with the same MRI scanner before, wherein the respective features in the training samples were analysed manually using a typical software for image analysis. It was found that reliable and fast identification of the respective features is possible using the method of the present invention.

### Reference Signs

- 12: process step a)
- 14: process step b)
- 16: process step c)
- 18: process step d)

- 100: MRI system
- 102: industrial samples
- 104: conveyor
- 106: MRI scanner

- 200: inference module
- 202: memory
- 204: machine learning module
- 206: processor

- 300: undersampled MRI data
- 302: training set
- 304: first class
- 306: second class
- 308: third class

## Claims

1. Method for automated non-invasive identification of a predetermined feature in a multitude of industrial samples (102) of a predefined sample type, the method comprising the steps of:
a) conveying an industrial sample (102) of the predefined sample type into an MRI scanner (106),
b) recording in an MRI measurement for at least one slice or at least a partial volume of the industrial sample undersampled MRI data (300), comprising:
- undersampled raw MRI data, comprising a multitude of time dependent signals for different phases, and/or
- processed MRI data, obtained from processing undersampled raw MRI data, and
c) analysing the undersampled MRI data (300) with an inference module (200) for identifying a predetermined feature of the industrial sample (102) using a machine learning module (204) that is trained for identifying the predetermined feature in industrial samples (102) of the predefined sample type from undersampled MRI data (300),
wherein the inference module (200) comprises a memory (202) storing the machine learning module (204) and a processor (206) for controlling the inference module (200), wherein the inference module (200) is configured to provide the undersampled MRI data (300) as an input to the machine learning module (200) and to analyse the undersampled MRI data (300) using the machine learning module (204), wherein the machine learning module (204) is trained for identifying the predetermined feature in industrial samples (102) of the predefined sample type using a training set (302) comprising undersampled MRI data (300) of different training samples of the predefined sample type, wherein a fraction of the training samples comprises the predetermined feature and a fraction of the training samples does not comprise the predetermined feature.

2. Method according to claim 1, further comprising the step of:
d) classifying the industrial sample (102) based on the result of the analysis, wherein the classification is preferably conducted by the inference module (200), wherein the inference module (200) is preferably configured to classify industrial samples (102) of the predefined type based on the result of the identification of the predetermined feature, wherein the multitude of industrial samples (102) is most preferably conveyed out of the MRI scanner (106) and sorted based on the classification.

3. Method according to any one of claims 1 or 2, wherein the undersampled MRI data (300), comprises undersampled raw MRI data, wherein the undersampled MRI data (300) preferably consists of undersampled raw MRI data.

4. Method according to any one of claims 1 to 3, wherein the undersampled MRI data (300), comprises processed MRI data, wherein the undersampled MRI data (300), preferably consists of processed MRI data.

5. Method according to any one of claims 1 to 4, wherein the processed MRI data is obtained as an MRI image by Fourier transforming or otherwise linearly or nonlinearly encoding the undersampled MRI raw data along an undersampled frequency-encoding dimension and an undersampled phase-encoding dimension, wherein the MRI image comprises at least one aliasing artefact, wherein the machine learning module (204) is trained for identifying the predetermined feature in industrial samples (102) of the predefined sample type from MRI images that comprise at least one aliasing artefact.

6. Method according to any one of claims 1 to 5, wherein the method is operated at a rate of 1000 or more industrial samples (102) per hour, preferably 5000 or more industrial samples (102) per hour.

7. Method according to any one of claims 1 to 6, wherein the method is sequentially applied to a multitude of industrial samples (102) and/or wherein the method is simultaneously applied to a multitude of industrial samples (102), wherein preferably the method is sequentially applied several times simultaneously to a multitude of industrial samples (102).

8. Method according to any one of claims 1 to 7, wherein the method identifies the presence, absence or magnitude of the predetermined feature and/or wherein subsequent processing steps of the industrial sample (102) are controlled in dependence of the presence, absence or magnitude of the predetermined feature.

9. Method according to any one of claims 1 to 8, wherein the predefined sample type is selected from the group consisting of animal products, plants, and products derived from these materials, preferably meat, fish meat, eggs, fruits, seeds and processed food and drinks, more preferably eggs, seeds, nuts and chocolate products.

10. Method according to any one of claims 1 to 9, wherein the predetermined feature is selected from the group consisting of chemical composition, physical properties, in particular magnetic properties, and structural features, preferably structural features, more preferably anatomical features, biological features, morphological dimensions, sample structure, spatial distribution of elements in the industrial sample and presence of impurities.

11. Method according to any one of claims 1 to 10, wherein the machine learning module (204) is trained for identifying the predetermined feature in industrial samples (102) of the predefined sample type using the training set (302), wherein each undersampled MRI data (300) of different training samples is linked with information about the MRI Scanner (106) and/or the experimental parameters of the MRI measurement used to obtain the undersampled MRI data (300), so that the machine learning module (204) is trained for identifying the predetermined feature in industrial samples (102) of the predefined sample in a multitude of different MRI scanners (106) and/or under different experimental conditions, wherein the inference module (200) is configured to provide information about the MRI scanner (106) and/or the experimental parameters of the MRI measurement of the method as an input to the machine learning module (204), wherein the experimental parameters are preferably selected from the group comprising pulse lengths, pulse sequence, evolution times, repetitions times, sampling rate, phase increments, temperature and number of scans, or
wherein the training set (302) comprises undersampled MRI data (300) of different training samples of the predefined sample type that was recorded with the same type of MRI scanner used in the method, wherein preferably similar, more preferably basically identical, experimental parameters were employed for recording the undersampled MRI data (300) of the training samples as are used in the method.

12. Method according to any one of claims 1 to 11, wherein the machine learning module (204) is a deep learning network or an artificial neural network, preferably a deep learning network.

13. Inference module (200) for analysing undersampled MRI data (300) of an industrial sample (102) of a predefined sample type, using a machine learning module (204), preferably in a method according to any one of claims 1 to 12,
wherein the inference module (200) comprises a memory (202) storing the machine learning module (204) and a processor (206) for controlling the inference module (200),
wherein the inference module (200) is configured to provide undersampled MRI data (300) as an input to the machine learning module (204) and to analyse the undersampled MRI data (300) using the machine learning module (204),
wherein the machine learning module (204) is trained for identifying the predetermined feature in industrial samples (102) of the predefined sample type using a training set (302) comprising undersampled MRI data (300) of different training samples of the predefined sample type, wherein a fraction of the training samples comprises the predetermined feature and a fraction of the training samples does not comprise the predetermined feature.

14. MRI system (100) for conducting the method according to any one of claims 1 to 12, comprising:
a) an MRI scanner (106) for obtaining undersampled MRI data (300) of industrial samples (102) of a predefined sample type,
b) a conveyor (104) for conveying a multitude of industrial samples (102) into the MRI scanner (106), and
c) an inference module (200) according to claim 13, that is connected to the MRI scanner (106).

15. Computer program product comprising instructions which, when the program is executed by a computer, preferably by an inference module (200) according to claim 13, cause the computer to carry out step c) of the method according to any one of claims 1 to 12.
